(19) Europäisches Patentamt
European Patent Office
Office européen des brevets

(11) Numéro de publication : **0 113 600**
**B1**

(12) **FASCICULE DE BREVET EUROPÉEN**

(45) Date de publication du fascicule du brevet : **29.07.87**

(21) Numéro de dépôt : **83402141.2**

(22) Date de dépôt : **04.11.83**

(51) Int. Cl.⁴ : **C 07 D405/04**, C 07 D405/14, C 07 D413/14, A 61 K 31/44// C07D213/57, C07D405/12

(54) **Dérivés de la gamma-butyrolactone protecteurs du myocarde présentant une activité antiarythmique, procédé pour leur préparation et médicaments en contenant.**

(30) Priorité : **08.11.82 FR 8218705**

(43) Date de publication de la demande : **18.07.84 Bulletin 84/29**

(45) Mention de la délivrance du brevet : **29.07.87 Bulletin 87/31**

(84) Etats contractants désignés : **AT BE CH DE FR GB IT LI LU NL SE**

(56) Documents cités :
**EP-A- 0 027 412**
**CHEMISCHE BERICHTE, vol. 114, no. 1, 9 janvier 1981, Verlag Chemie GmbH, Weinheim (DE) H. STAMM et al.: "Einstufensynthese von Pyrrolidonen durch Amidoethylierung einfacher Ester mit N-acylaziridinen", pages 32-48.**

(73) Titulaire : **SANOFI, société anonyme**
**40, Avenue George V**
**F-75008 Paris (FR)**

(72) Inventeur : **Bernhart, Claude**
**144 rue des Muriers**
**F-34980 Saint-Gely du Fesc (FR)**
Inventeur : **Cautreels, Werner**
**5 Impasse des Décurions**
**F-34170 Casteinau-le-Lez (FR)**
Inventeur : **Gautier, Patrick**
**Manavielle**
**F-34660 Cournonterral (FR)**

(74) Mandataire : **Gillard, Marie-Louise et al**
**Cabinet Beau de Loménie 55, Rue d'Amsterdam**
**F-75008 Paris (FR)**

**EP 0 113 600 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne en tant que produits industriels nouveaux des dérivés de la γ-butyrolactone ainsi que leurs méthodes de préparation et leur application en thérapeutique.

Ces dérivés qui sont protecteurs du myocarde et présentent une activité antiarythmique sont substitués par un groupe pyridine.

Dans le brevet EP 27 412, on a déjà décrit des dérivés de la pyridine à titre de protecteurs du myocarde ayant une activité antiarythmique. Ces dérivés sont des pyridines substituées par un groupe alkyle portant une fonction amide et une fonction amine.

Au contraire, les composés de l'invention peuvent être considérés, ainsi que le montre la formule générale ci-après, comme étant des pyridines portant un cycle butyrolactone substitué par un groupe alkyle amino.

Les nouveaux composés selon l'invention répondent à la formule générale :

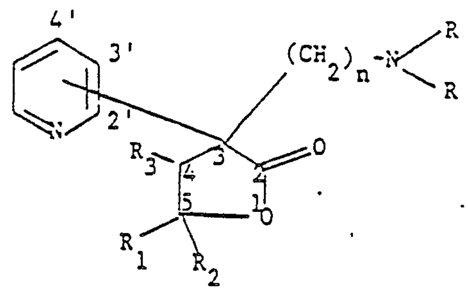

(I)

dans laquelle :

R représente un groupe alkyle droit ou ramifié ayant de 2 à 5 atomes de carbone ou encore le groupe

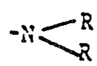

représente un groupe morpholino ou pipéridino éventuellement substitué par 1 à 4 groupes méthyle ;

n = 2 ou 3 ;

$R_1$ et $R_2$ considérés indépendamment représentent chacun l'hydrogène ou un groupe alkyle ayant de 1 à 4 atomes de carbone,

ou $R_1$ et $R_2$ pris ensemble représentent un groupe $(CH_2)_m$ où m = 4 ou 5, dans ces deux cas $R_3$ étant H ou encore $R_1$ représente H et $R_2$ et $R_3$ pris ensemble représentent $(CH_2)_p$ où p est 3 ou 4 ;

Le cycle butyrolactone substitue le groupe pyridyle en 2', 3' ou 4'.

Les composés (I) fournissent avec les acides minéraux ou organiques des sels solubles. Ces sels, avec des acides pharmaceutiquement acceptables, font partie intégrante de l'invention.

Les composés (I) possèdent toujours un atome de carbone asymétrique, à savoir l'atome 3 du cycle de la γ-butyrolactone. Si les substituants $R_1$ et $R_2$ sont différents, l'atome de carbone 5 qui les porte est aussi un atome de carbone asymétrique. Par suite, les composés (I) peuvent exister sous la forme de diastéréoisomères et d'isomères optiques. Ces isomères ainsi que leurs mélanges appartiennent à l'invention.

Les composés selon l'invention où $R_3$ est H sont obtenus à partir d'un pyridylacétonitrile selon le schéma réactionnel indiqué ci-après :

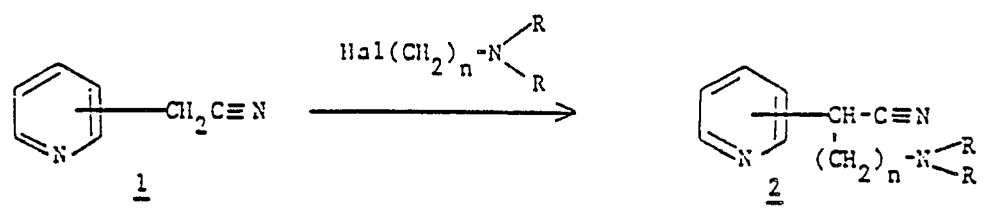

1 2

(Voir schéma p. 3)

(Suite)

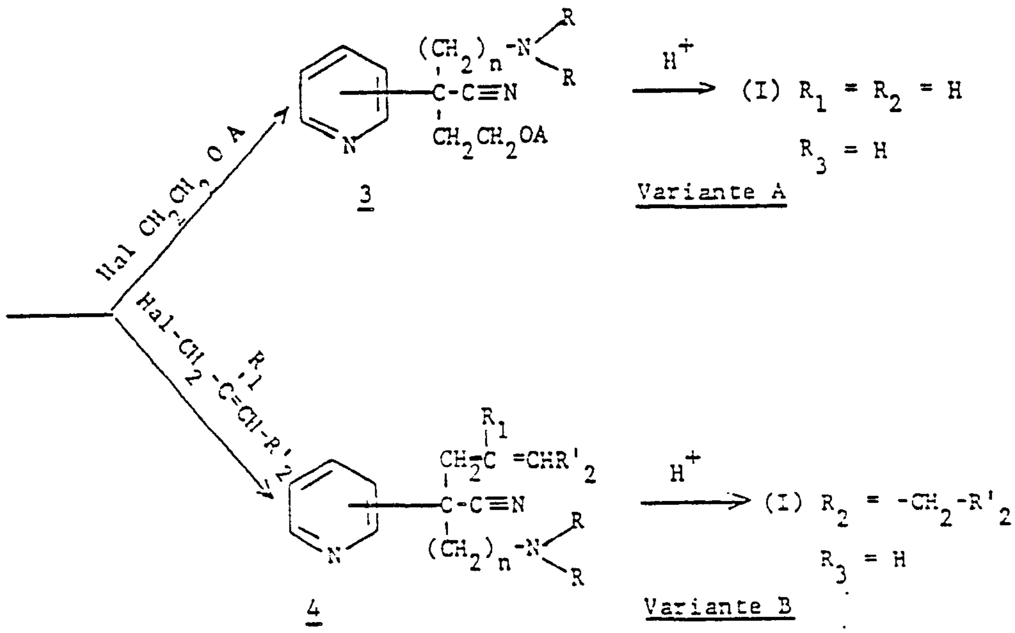


3

4

Le pyridylacétonitrile 1 est soumis tout d'abord à une réaction d'alkylation par un composé

$$\text{Hal}-(CH_2)_n-N\langle^{R}_{R}$$

(Hal représentant un halogène), en présence d'une base organique ou minérale pour conduire au composé 2. A partir de celui-ci, deux variantes sont possibles :

Variante A :

Le composé 2 est à nouveau substitué par un composé Hal—$CH_2CH_2OA$ dans lequel Hal représente un halogène et A représente un groupe protecteur de la fonction hydroxyle facilement éliminable en milieu acide et notamment un groupe tétrahydropyrannyle.

On opère dans un solvant inerte tel qu'un hydrocarbure benzénique en présence d'hydrure de sodium, à une température comprise entre 80 et 120 °C.

Le composé 3 ainsi obtenu est traité par un acide minéral concentré tel que l'acide phosphorique à 85 % pour conduire au composé (I) où $R_1 = R_2 = H$.

Variante B :

On substitue à nouveau le composé 2 par un dérivé insaturé

$$\text{Hal}-CH_2-\overset{R_1}{C}=CH-R'_2,$$

dans lequel Hal représente un halogène et $R'_2$ représente l'hydrogène ou un groupe alkyle en $C_1$-$C_3$, ou encore $R_1 + R'_2$ représente un groupe $(CH_2)_{m'}$ avec m' = 3 ou 4.

La réaction s'effectue au sein d'un solvant tel que le diméthylformamide en présence d'amidure de sodium, à une température comprise entre 20 et 80 °C.

Le composé 4 ainsi obtenu est traité par un acide minéral concentré et notamment par l'acide sulfurique concentré pour conduire au composé (I) dans lequel le substituant $R_2$ représente $CH_2R'_2$.

Lorsque $R_1$ représente l'hydrogène et que $R_2 + R_3$ représentent ensemble un groupe $(CH_2)_p$, les composés (I) peuvent être obtenus selon le schéma réactionnel

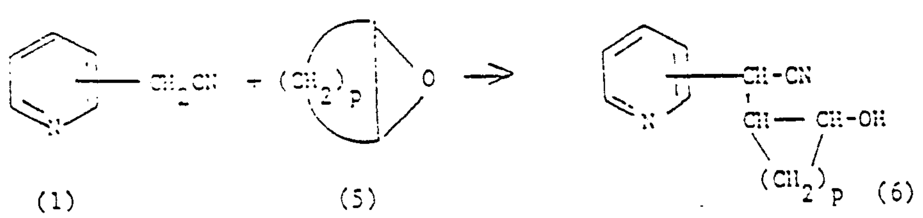

(1) (5) (6)

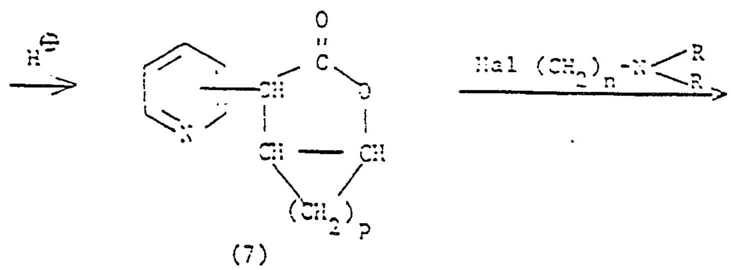


(7) (I)

L'ouverture de l'époxyde (5) par l'anion du pyridylacétonitrile (1) conduit à l'hydroxy nitrile (6).

Pour obtenir l'anion du pyridylacétonitrile, il convient d'utiliser le diisopropylamidure de lithium (préparé in situ par action de la diisopropylamine sur le butyllithium) au sein d'un solvant inerte, tel que le tétrahydrofuranne et à basse température (— 60 à — 80 °C). A partir de l'hydroxynitrile (6), on passe à la lactone (7) par hydrolyse en milieu acide. On utilise un acide minéral concentré tel que l'acide phosphorique à 85 %. Enfin, on obtient le composé (I) par substitution du composé 7 avec un dérivé de formule

$$Hal(CH_2)_n-N\begin{smallmatrix}R\\R\end{smallmatrix}$$

(dans laquelle Hal est un halogène) en présence d'un agent de sodation tel que l'hydrure de sodium, au sein d'un solvant tel que le tétrahydrofuranne.

Les sels des composés (I) s'obtiennent par les méthodes habituelles de salification.

Quand $R_1$ et $R_2$ sont différents, les produits (I) existent sous la forme de diastéréoisomères. Le procédé selon l'invention conduit à un mélange de diastéréoisomères. Ce mélange peut être utilisé tel quel ou bien séparé en ses constituants par les méthodes habituelles et, en particulier, chromatographie.

Les exemples suivants, nullement limitatifs, sont donnés à titre d'illustration pour la préparation des composés selon l'invention.

## Exemple 1

(Diisopropylamino-2 éthyl)-3 (pyridyl-2)-3 γ-butyrolactone, phosphate. (SR 41340)

$$R = -CH\begin{smallmatrix}CH_3\\CH_3\end{smallmatrix} \quad ; \quad R_1 = R_2 = R_3 = H; \; n = 2 \qquad (I)$$

a) Diisopropylamino-4 (pyridyl)-2)-2 butyronitrile.

On mélange 8 g de pyridyl-2 acétonitrile, 8,8 g de chloro-1 diisopropylamino-2 éthane et 0,27 g de chlorure de benzyltriméthylammonium, puis, en maintenant la température en dessous de 35 °C, on ajoute 35 ml de solution de soude à 50 %. On chauffe le mélange à 35 °C pendant 5 h. Après refroidissement, on dilue avec de l'eau et extrait avec de l'éther. On sépare la phase organique et on la sèche sur sulfate de sodium, puis évapore le solvant à siccité.

Par distillation du résidu, on obtient une huile jaune (9,36 g) ; E./8 Pa (0,06 mm Hg) : 132-134 °C.

b) (Diisopropylamino-2 éthyl)-2 (pyridyl-2)-2 (tétrahydropyranyloxy-2)-4 butyronitrile.

On porte à reflux pendant 18 h le mélange de 9,3 g du nitrile obtenu ci-dessus, 7,5 g de chloro-1 (tétrahydropyranyloxy-2)-2 éthane et 1,75 g d'hydrure de sodium dans 200 ml de toluène. Après refroidissement, on ajoute de l'eau, puis décante la phase organique. On sèche sur sulfate de sodium et évapore le solvant à siccité.

On obtient une huile (14 g) utilisée telle quelle pour l'opération suivante.

c) SR 41340.

On dissout le produit obtenu au paragraphe b) dans 70 ml d'acide phosphorique à 85 % et chauffe le mélange à 90-100 °C pendant 1 h. On verse sur de la glace et extrait de l'éther. On sépare la phase organique et alcalinise la phase aqueuse avec une solution de carbonate de potassium. On extrait à nouveau avec de l'éther et réunit les extraits organiques. On sèche sur sulfate de sodium, puis évapore le solvant à siccité et distille le résidu sous pression réduite.

On obtient une huile (7,5 g) ; E./13,33 Pa (0,1 mm Hg) : 149-152 °C.

Phosphate :

On dissout 5,5 g du produit ci-dessus dans 50 ml d'éthanol et ajoute 2,18 g d'une solution d'acide phosphorique à 85 %. On évapore, reprend le résidu dans l'éther et laisse cristalliser. On essore le solide et lave avec de l'éther.

Poids : 5,95 g ; F. : 107-109 °C.

Exemple 2

Cyclohexanespiro-5 (diisopropylamino-2 éthyl)-3 (pyridyl-2)-3 γ-butyrolactone. (SR 41412)

$$R = -CH(CH_3)_2 \; ; \; n = 2; \; R_1+R_2 = (CH_2)_5; \; R_3 = H \qquad (I)$$

a) (Cyclohexényl-1 méthyl)-2 diisopropylamino-4 (pyridyl-2)-2 butyronitrile.

Dans un tricol, on introduit sous atmosphère d'azote 1,45 g d'hydrure de sodium et 20 ml de diméthylformamide. On ajoute goutte à goutte la solution de 12,25 g de diisopropylamino-4 (pyridyl-2)-2 butyronitrile (exemple 1 a)), dans 20 ml de diméthylformamide, puis, après la fin de l'introduction, on agite pendant 1 h à température ambiante.

On ajoute alors 9,6 g de bromométhyl-1 cyclohexène dissous dans 20 ml de diméthylformamide et agite à nouveau pendant 2 h à température ambiante. On évapore le solvant sous vide et on reprend le résidu par l'eau et l'éther. On sépare la couche organique et réextrait la phase aqueuse avec de l'éther. On réunit les extraits éthérés, sèche sur sulfate de sodium et évapore le solvant à siccité.

On obtient 18 g de produit brut utilisé tel quel pour l'étape suivante.

b) SR 41412.

On dissout le composé obtenu ci-dessus dans 150 ml d'acide phosphorique à 85 % et chauffe à 130 °C pendant 3 h.

On verse le mélange réactionnel sur de la glace et ajoute de l'éther, puis on alcalinise la phase aqueuse par de la solution de soude à 40 % en refroidissant pour que la température reste inférieure à 20 °C.

On sépare la couche éthérée et réextrait avec de l'éther. On sèche les extraits éthérés sur sulfate de sodium et évapore le solvant à siccité.

Le résidu (8,7 g) est chromatographié sur colonne d'alumine. En éluant avec le mélange pentane-acétate d'éthyle 97-3 vol/vol, on obtient 3,1 g du produit attendu qui cristallise. F. : 42-44 °C.

Exemple 3

(Diisopropylamino-2 éthyl)-3 méthyl-5 (pyridyl-2)-3 γ-butyrolactone phosphate. (SR 41653)

$$R = -CH(CH_3)_2 \; ; \; n = 2; \; R_2 = CH_3; \; R_1 = R_3 = H \qquad (I)$$

Mélange des diastéréoisomères.

On opère comme dans l'exemple 2 à partir du même produit de départ mais en remplaçant dans l'étape a) le bromométhyl-1 cyclohexène par une quantité équivalente de bromure d'allyle. La cyclisation s'effectue comme dans l'exemple 2 b) par chauffage à 150 °C pendant 6 h. Le produit brut ainsi obtenu est distillé sous pression réduite.

É./20 Pa (0,15 mm Hg) : 120-124 °C.

Le phosphate est préparé comme dans l'exemple 1 c). F : 157-159 °C.

Exemple 4

Diméthyl-5,5 (di-sec-butylamino-2 éthyl)-3 (pyridyl-2)-3 γ-buryrolactone. (SR 41652)

$$R = -CH(CH_3)-CH_2CH_3; \; n = 2; \; R_1 = R_2 = CH_3; \; R_3 = H \qquad (I)$$

Le produit de départ est préparé comme dans l'exemple 1 a), en remplaçant le chloro-1 diisopropylamino-2 éthane par une quantité équivalente de chloro-1 di-sec-butylamino-2 éthane. É./13,33 Pa (0,1 mm Hg) : 120-125 °C.

A partir de ce produit, on opère comme dans l'exemple 2 a), en remplaçant le bromométhyl-1

cyclohexène par une quantité équivalente de chloro-3 méthyl-2 propène.

Le produit brut ainsi obtenu est cyclisé comme dans l'exemple 2b). Après chromatographie sur colonne d'alumine en éluant avec le mélange pentane-acétate d'éthyle 95-5 vol/vol, on obtient le produit attendu. F. : 67-68 °C.

## Exemple 5

(Diisopropylamino-2 éthyl)-3 diméthyl-5,5 (pyridyl-2)-3 γ-butyrolactone. (SR 41098)

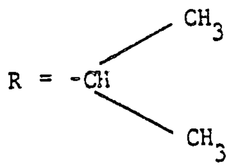 ; n = 2; $R_1 = R_2 = CH_3$; $R_3 = H$ (I)

A partir du produit de départ de l'exemple 1 a), on opère comme dans l'exemple 4 avec le chloro-3 méthyl-2 propène. Le produit brut ainsi obtenu est cyclisé par chauffage à 50 °C pendant 30 min avec de l'acide sulfurique concentré (d : 1,83). On traite comme dans l'exemple 2 b) et, après chromatographie sur alumine en éluant avec le mélange pentane-acétate d'éthyle 90-10 vol/vol, on obtient le produit attendu. F. : 58-59 °C.

## Exemples 6 à 21

En opérant comme dans l'exemple 1 a), mais en faisant varier le dérivé halogéné utilisé, on obtient divers nitriles substitués (2). Par action sur ceux-ci d'un dérivé insaturé convenablement choisi et en opérant comme dans les exemples 2, 4 et 5, on prépare les composés (4) correspondants qui sont directement cyclisés en composé (I) soit par l'acide phosphorique selon l'exemple 4, soit par l'acide sulfurique suivant l'exemple 5. Les produits (I) ainsi préparés sont réunis dans le tableau 1 ci-après.

(Voir Tableau pages suivantes)

Tableau 1

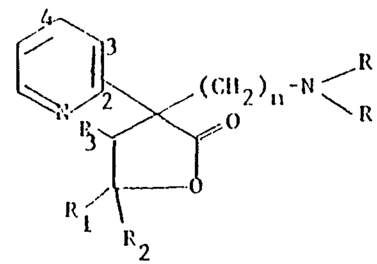

| Exemple n° | n° code produit | Position substitution pyridine | n | 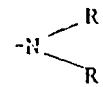 | $R_1$ | $R_2$ | $R_3$ | Cyclisation | Base ou sel F, °C (solvant) |
|---|---|---|---|---|---|---|---|---|---|
| 6 | SR 41696 | 2 | 2 | 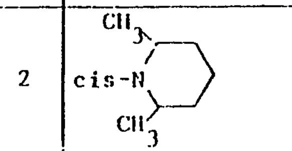 | $-CH_3$ | $-CH_3$ | H | $SO_4H_2$ | Base F : 58-59 |
| 7 | SR 41816 | 2 | 3 | $-N[CH-(CH_3)_2]_2$ | $-CH_3$ | $-CH_3$ | H | $SO_4H_2$ | Dichlorhydrate, $1H_2O$ F : 158-160 (isopropanol) |
| 8 | SR 41913 | 2 | 2 | 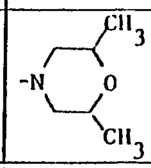 | $-CH_3$ | $-CH_3$ | H | $SO_4H_2$ | Dichlorhydrate, F : 201-202 (acétone) |
| 9 | SR 41914 | 2 | 2 | 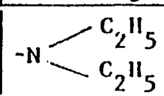 | $-CH_3$ | $-CH_3$ | H | $PO_4H_3$ | Dichlorhydrate, 0,5 $H_2O$ F : 140-142 (isopropanol) |
| 10 | SR 41941 | 2 | 2 | 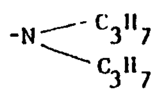 | $-CH_3$ | $-CH_3$ | H | $SO_4H_2$ | Oxalate F : 103-105 (acétone) |
| 11 | SR 41942 | 2 | 2 | $-N[CH(CH_3)_2]_2$ | $-C_2H_5$ | $-C_2H_5$ | H | $PO_4H_3$ | Oxalate F : 128-130 (éther) |

Tableau 1 (Suite)

| Exemple n° | n° code produit | Position substitution pyridine | n | 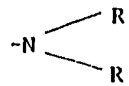 | $R_1$ | $R_2$ | $R_3$ | Cyclisation | Base ou sel F. °C (solvant) |
|---|---|---|---|---|---|---|---|---|---|
| 12 | SR 41943 | 2 | 2 | 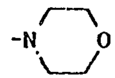 | $-CH_3$ | $-CH_3$ | H | $PO_4H_3$ | Oxalate F : 188-189 (éthanol) |
| 13 | SR 41944 | 2 | 2 | 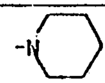 | $-CH_3$ | $-CH_3$ | H | $PO_4H_3$ | Base F : 55-57 (hexane) |
| 14 | SR 42167 | 2 | 2 | 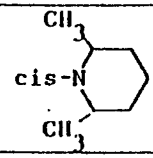 | $(CH_2)_5$ | | H | $PO_4H_3$ | Chlorhydrate, 0,75 $H_2O$ F : 135-137 (butanone-2) |
| 15 | SR 42180 | 2 | 2 | 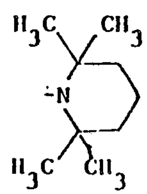 | $-CH_3$ | $-CH_3$ | H | $PO_4H_3$ | Base F : 87-88 (éthanol-eau) |
| 16 | SR 42205 | 2 | 2 | $-N[CH(CH_3)_2]_2$ | $-C_3H_7$ | $-C_3H_7$ | H | $PO_4H_3$ | Oxalate F : 121-123 (acétone) |
| 17 | SR 42206 | 3 | 2 | 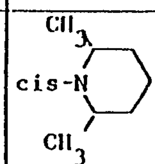 | $-CH_3$ | $-CH_3$ | H | $PO_4H_3$ | Base (liquide) |
| 18 | SR 42298 | 2 | 2 | " | $-C_2H_5$ | $-C_2H_5$ | H | $PO_4H_3$ | Oxalate, 0,75 $H_2O$ F : 80-82 (acétate d'éthyle) |

Tableau 1 (Suite)

| Exemple n° | n° code produit | Position substitution pyridine | n | 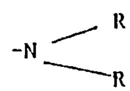 | $R_1$ | $R_2$ | $R_3$ | Cyclisation | Base ou sel F. °C (solvant) |
|---|---|---|---|---|---|---|---|---|---|
| 19 | SR 42408 | 2 | 2 | cis-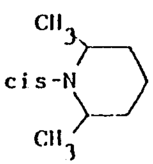 | $-C_3H_7$ | $-C_3H_7$ | H | $PO_4H_3$ | Dichlorhydrate F : 189-190 (isopropanol) |
| 20 | SR 42435 | 2 | 3 | " | $-CH_3$ | $-CH_3$ | H | $PO_4H_3$ | Phosphate F : 165-167 (isopropanol) |
| 21 | SR 42483 | 2 | 2 | trans-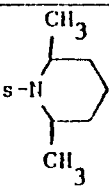 | $-CH_3$ | $-CH_3$ | H | $SO_4H_2$ | Base (liquide) |

Exemple 22

[(cis-diméthyl-2,6 pipéridino)-2 éthyl]-3 (pyridyl-2)-3 hexahydro-trans-benzofurannone-2 (3H) (SR 42455)

$R_1 = H$; $R_2 + R_3 = (CH_2)_4$; 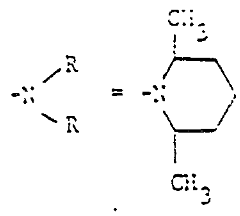 cis (I)

a) α-(trans-hydroxy-2 cyclohexyl) pyridyl-2 acétonitrile.

Au mélange de 45,45 g de diisopropylamine et 200 ml de tétrahydrofuranne anhydre refroidi à — 20 °C, on ajoute sous atmosphère d'azote en agitant 300 ml d'une solution 1,6 M de butyllithium dans l'hexane. On refroidit ensuite à — 70 °C, puis on ajoute la solution de 53,1 g de pyridyl-2 acétonitrile dans 200 ml de tétrahydrofuranne anhydre. On agite pendant 15 min à température inférieure ou égale à — 70 °C, puis on ajoute à la même température la solution de 48,5 g d'époxycyclohexane dans 200 ml de tétrahydrofuranne anhydre. On laisse remonter la température jusqu'à température ambiante (environ 20 °C) et agite le mélange pendant 15 h à cette température.

On refroidit par un bain de glace et ajoute 300 ml d'eau. On décante la phase organique puis réextrait la phase aqueuse deux fois avec de l'éther.

On réunit les phases organiques, lave deux fois avec une solution saturée de chlorure de sodium, puis sèche la solution sur sulfate de sodium. On évapore les solvants à siccité et cristallise le résidu dans l'isopropanol.

On obtient 74,6 g du produit attendu. F. : 110-112 °C.

b) (Pyridyl-2)-3 hexahydro-trans-benzofurannone-2 (3H).

On chauffe à 90 °C pendant 30 min le mélange de 32,4 g du produit obtenu ci-dessus et 160 ml d'acide phosphorique à 85 %. On verse le mélange réactionnel sur de la glace, puis on alcalinise par addition d'une solution de soude à 30 % en maintenant la température au-dessous de 25 °C.

On extrait avec de l'acétate d'éthyle et sèche la solution sur sulfate de sodium. On évapore le solvant à siccité et reprend le résidu solide dans de l'éther isopropylique. On essore le solide et obtient le produit attendu (31 g). F : 124-125 °C.

c) SR 42455.

Dans un ballon, sous atmosphère d'azote, on introduit 100 ml de tétrahydrofuranne anhydre et 2,5 g d'une suspension à 55 % dans l'huile d'hydrure de sodium. On chauffe au reflux, puis on ajoute goutte à goutte la solution dans 250 ml de tétrahydrofuranne anhydre de 10,85 g du produit obtenu ci-dessus et 917 g de (chloro-2 éthyl)-1 cis-diméthyl-2,6 pipéridine. Après la fin de l'addition, on maintient au reflux pendant 1 h, puis on évapore le tétrahydrofuranne. On reprend le résidu dans l'eau et extrait avec de l'éther. On sèche la solution sur sulfate de sodium, puis on évapore le solvant et chromatographie le résidu huileux sur une colonne d'alumine.

En éluant avec un mélange pentane-acétate d'éthyle 95-5 (volume/volume), on obtient une huile (6,7 g).

On ajoute du pentane et laisse cristalliser lentement à 0 °C. On essore le solide et lave avec un peu de pentane refroidi à 0 °C. On obtient finalement des cristaux (3,5 g). F : 81-82 °C.

Les produits de l'invention ont été étudiés en pharmacologie, en particulier en vue de mettre en évidence leurs propriétés antiarythmiques.

Protocole :

Le pouvoir antiarythmique de ces molécules a été apprécié sur un modèle animal d'arythmie ventriculaire.

Des chiens batards sont anesthésiés puis soumis à la mise en place, par cathétérisme rétrograde, d'une spire métallique dans le lit coronarien. Dans le même temps, un micro-émetteur modulateur de fréquence est fixé au dos de l'animal et relié à deux électrodes précordiales.

L'animal, remis dans son box, accuse alors une thrombose progressive de l'artère interventriculaire antérieure. Ainsi se constitue un infarctus myocardique localisé et transmural, générateur d'une activité électrique anormale mais répétitive : tachycardie ventriculaire.

Dans cet état, 24 h après la pose de la spire, les drogues sont administrées per os et le système télémétré permet de suivre en temps réel l'évolution de la disrythmie du chien vigile.

Un comptage des complexes systoliques sinusaux et pathologiques est assuré en permanence par

des procédés électroniques. Ainsi peut-on quantifier la qualité et la durée d'action du produit. Le comportement de l'animal est observé.

Résultats :

Un produit est considéré comme actif s'il supprime au moins 60 % des complexes anormaux ou s'il induit un rythme sinusal.

Les résultats obtenus avec divers produits de l'invention figurent dans le tableau 2.

Tableau 2

| Produit n° de code SR | Dose mg/kg per os | Nombre d'animaux | Durée d'action |
|---|---|---|---|
| 41098 | 50 | 2 | 3 h |
| 41412 | 50 | 1 | 1 h |
| 41652 | 50 | 1 | 1 h 50 |
| 41653 | 50 | 1 | 1 h 25 |
| 41696 | 20 | 1 | supérieure à 24 h |
| | 5 | 1 | 3 h 30 |
| | 3 | 1 | 2 h |
| 41816 | 50 | 2 | supérieure à 24 h |
| 41913 | 50 | 1 | supérieure à 24 h |
| 41942 | 50 | 1 | supérieure à 24 h |
| 42180 | 50 | 1 | 8 h |

Ces résultats montrent que les produits selon l'invention sont doués d'une forte activité sur la dysrythmie.

Par ailleurs, les composés selon l'invention sont relativement peu toxiques et, en particulier, ne présentent aucun signe de toxicité aux doses où ils manifestent leur activité antiarythmique.

Par suite, les produits (I) peuvent être utilisés en thérapeutique humaine comme protecteur du myocarde pour la correction de troubles du rythme ventriculaire d'origine ischémique.

Les produits pourront être présentés sous les formes galéniques correspondant à la voie orale (comprimés, gélules, ...) et à la voie parentérale (ampoules injectables).

La dose nécessaire à la restauration du rythme sinusal chez l'homme est comprise entre 5 et 150 mg par voie intraveineuse et entre 40 et 800 mg par voie orale, par jour, environ.

A titre d'exemple, on indique la préparation galénique suivante :

| Comprimés | |
|---|---|
| SR 41696 | 0,200 g |
| Cellulose microcristalline | 0,140 g |
| Lactose | 0,140 g |
| Stéarate de magnésium | 0,020 g |
| | 0,500 g |

**Revendications** (pour les Etats contractants : BE, CH, DE, FR, GB, IT, LI, LU, NL, SE)

1. Dérivés de la γ-butyrolactone de formule

(Voir dessin page 12)

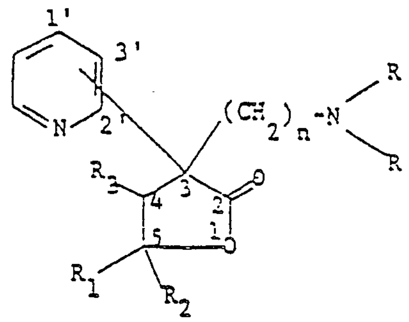

(I)

dans laquelle

R est un groupe alkyle droit ou ramifié ayant de 2 à 5 atomes de carbone ou le groupe

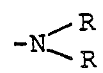

représente un groupe morpholino ou pipéridino éventuellement substitué par 1 à 4 groupes méthyle ;

n = 2 ou 3 ;

$R_1$ et $R_2$ considérés indépendamment représentent chacun l'hydrogène ou un groupe alkyle ayant de 1 à 4 atomes de carbone ; ou $R_1$ et $R_2$ pris ensemble représentent un groupe $(CH_2)_m$ où m = 4 ou 5, et $R_3$ est H ou encore $R_1$ est H et $R_2$ et $R_3$ pris ensemble représentent $(CH_2)_p$ où p est 3 ou 4.

le cycle butyrolactone étant accroché au cycle pyridyle en position 2', 3' ou 4',

et les sels, isomères et mélanges d'isomères desdits produits.

2. Dérivé selon la revendication 1, caractérisé en ce que $R_1$ et $R_2$ représentent chacun le groupe méthyle, $R_3$ est l'hydrogène,

n = 2 und 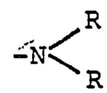

est le groupe,

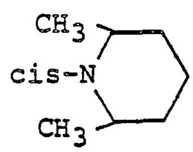

le cycle butyrolactone étant accroché au cycle pyridyle en position 2' ou 3'.

3. Dérivé selon la revendication 1, caractérisé en ce que $R_1$ et $R_2$ ensemble représentent le groupe $-(CH_2)_5-$, $R_3$ est l'hydrogène, n = 2 et

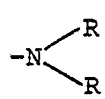

est le groupe,

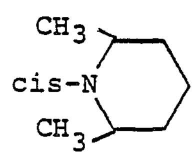

le cycle butyrolactone étant accroché au cycle pyridyle en position 2'.

4. Dérivé selon la revendication 1, caractérisé en ce que $R_1$ et $R_2$ représentent chacun le groupe éthyle, $R_3$ est l'hydrogène, n = 2 et

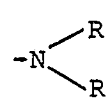

est le groupe,

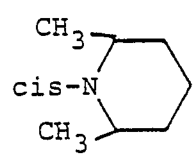

le cycle butyrolactone étant accroché au cycle pyridyle en position 2'.

5. Procédé de préparation des produits selon l'une quelconque des revendications 1 à 4, de formule (I) dans laquelle $R_3$ est H, caractérisé en ce que l'on utilise comme produit de départ le pyridylacétonitrile, que l'on fait réagir sur le composé de formule

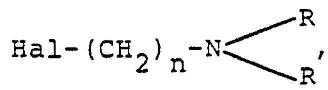

dans laquelle Hal est un halogène, n et R ayant les significations données à la revendication 1, la réaction étant effectuée en présence d'une base organique ; que l'on fait réagir le produit obtenu sur un composé choisi parmi les produits de formule Hal—$CH_2$—$CH_2$OA, dans laquelle Hal est un halogène et A un groupe protecteur de la fonction hydroxyle ou de formule

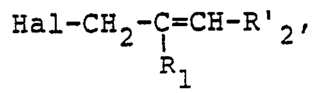

dans laquelle $R_1$ est H ou un groupe alkyle en $C_1$-$C_4$, $R'_2$ est H ou un groupe alkyle en $C_1$-$C_3$ ou $R_1$ et $R'_2$ forment ensemble un groupe —$(CH_2)_{m'}$ dans lequel m' = 3 ou 4, Hal est un halogène ; puis que l'on effectue la cyclisation du produit obtenu par un acide minéral concentré et qu'éventuellement, le composé de formule (I) ainsi obtenu est transformé en l'un de ses sels.

6. Procédé selon la revendication 5, dans lequel le réactif de la deuxième étape est Hal—$CH_2$—$CH_2$—OA, caractérisé en ce que cette deuxième étape est effectuée en solution dans un solvant inerte, en présence d'hydrure de sodium, à une température de 80 à 120 °C.

7. Procédé selon la revendication 5, dans lequel le réactif de la deuxième étape est,

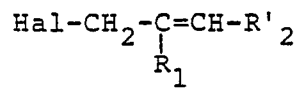

caractérisé en ce que la réaction est effectuée en solution dans un solvant en présence d'amidure de sodium.

8. Procédé selon la revendication 7, caractérisé en ce que la réaction est effectuée en solution dans le diméthylformamide.

9. Procédé de préparation des produits selon la revendication 1, de formule I, dans laquelle $R_1$ est H et $R_2$ et $R_3$ représentent ensemble le radical $(CH_2)_p$ dans lequel p est 3 ou 4, caractérisé en ce que l'on utilise comme produit de départ le pyridylacétonitrile anion que l'on fait réagir dans un solvant inerte et à basse température sur un époxyde de formule :

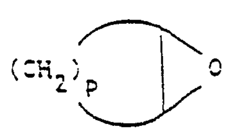

puis on réalise l'hydrolyse acide du produit hydroxylé obtenu et, enfin, on fait réagir le produit obtenu en solution et en présence d'un agent de sodation, avec un réactif de formule

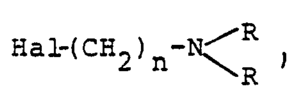

dans laquelle Hal est un halogène ; le produit de formule (I) ainsi obtenu est éventuellement transformé en l'un de ses sels.

10. Procédé selon la revendication 9, caractérisé en ce que l'hydrure de sodium est employé comme agent de sodation.

11. Médicaments présentant notamment un effet protecteur du myocarde, caractérisés en ce qu'ils contiennent au moins un produit selon l'une quelconque des revendications 1 à 4.

12. Médicaments selon la revendication 11, caractérisés en ce qu'ils contiennent de 5 à 800 mg dudit produit selon l'une quelconque des revendications 1 à 4.

**Revendications** (pour l'Etat contractant AT)

1. Procédé pour la préparation de dérivés de la γ-butyrolactone de formule

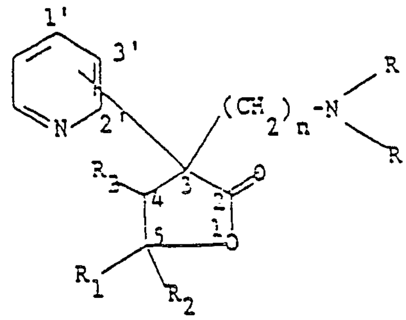

(I)

dans laquelle

R est un groupe alkyle droit ou ramifié ayant de 2 à 5 atomes de carbone ou le groupe

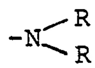

représente un groupe morpholino ou pipéridino éventuellement substitué par 1 à 4 groupes méthyle ;

n = 2 ou 3 ;

$R_1$ et $R_2$ considérés indépendamment représentent chacun l'hydrogène ou un groupe alkyle ayant de 1 à 4 atomes de carbone ; ou $R_1$ et $R_2$ pris ensemble représentent un groupe $(CH_2)_m$ où m = 4 ou 5, et $R_3$ est H ;

le cycle butyrolactone étant accroché au cycle pyridyle en position 2', 3' ou 4',

et les sels, isomères et mélanges d'isomères desdits produits, caractérisé en ce que l'on utilise comme produit de départ le pyridylacétonitrile, que l'on fait réagir sur le composé de formule

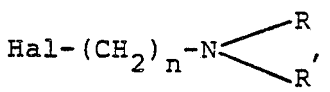

dans laquelle Hal est un halogène, n et R ayant les significations données ci-dessus, la réaction étant effectuée en présence d'une base organique ; que l'on fait réagir le produit obtenu sur un composé choisi parmi les produits de formule Hal—$CH_2$—$CH_2$—OA, dans laquelle Hal est un halogène et A un groupe protecteur de la fonction hydroxyle ou de formule

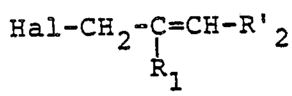

dans laquelle $R_1$ est H ou un groupe alkyle en $C_1$-$C_4$, $R'_2$ est H ou un groupe alkyle en $C_1$-$C_3$ ou $R_1$ et $R'_2$ forment ensemble un groupe —$(CH_2)_{m'}$ dans lequel m' = 3 ou 4, Hal est un halogène ; puis que l'on effectue la cyclisation du produit obtenu par un acide minéral concentré et, qu'éventuellement, le composé de formule (I) ainsi obtenu est transformé en l'un de ses sels.

2. Procédé selon la revendication 1 dans lequel le réactif de la deuxième étape est Hal—$CH_2$—$CH_2$—OA, caractérisé en ce que cette deuxième étape est effectuée en solution dans un solvant inerte, en présence d'hydrure de sodium, à une température de 80 à 120 °C.

3. Procédé selon la revendication 1, dans lequel le réactif de la deuxième étape est

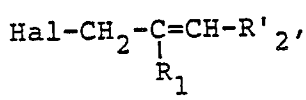

caractérisé en ce que la réaction est effectuée en solution dans un solvant, en présence d'amidure de sodium.

4. Procédé selon la revendication 3, caractérisé en ce que la réaction est effectuée en solution dans le diméthylformamide.

5. Procédé selon l'une des revendications 1 ou 4, caractérisé en ce que l'on fait réagir le pyridyl-2 ou pyridyl-3 acétonitrile avec un composé de formule

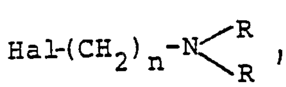

dans laquelle

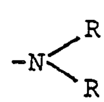

est le groupe

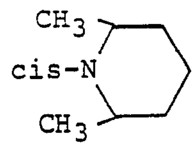

et n = 2 et en ce que l'on fait réagir le composé obtenu avec un composé de formule

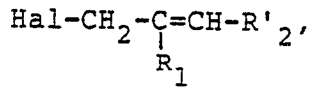

dans laquelle $R_1$ est $CH_3$ et $R'_2$ est l'hydrogène.

6. Procédé selon l'une des revendications 1 ou 4, caractérisé en ce que l'on fait réagir le pyridyl-2 acétonitrile avec un composé de formule

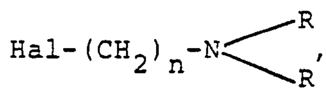

dans laquelle

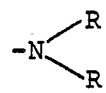

est le groupe

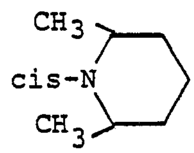

et n = 2 et en ce que l'on fait réagir le composé obtenu avec un composé de formule

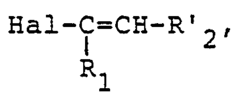

dans laquelle $R_1$ et $R'_2$ ensemble forment le groupe $—(CH_2)_4—$.

7. Procédé selon l'une des revendications 1 ou 4, caractérisé en ce que l'on fait réagir le pyridyl-2 acétonitrile avec un composé de formule

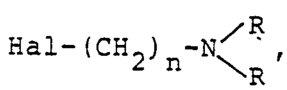

dans laquelle

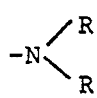

est le groupe

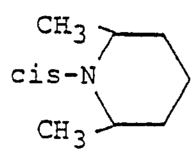

et n = 2 et en ce que l'on fait réagir le composé obtenu avec un composé de formule

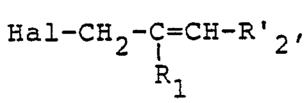

dans laquelle $R_1$ est le groupe éthyle et $R'_2$ est le groupe méthyle.

8. Procédé de préparation de dérivés de la γ-butyrolactone de formule (I) dans laquelle : R est un groupe alkyle droit ou ramifié ayant de 2 à 5 atomes de carbone ou le groupe

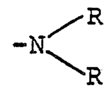

représente un groupe morpholino ou pipéridino éventuellement substitué par 1 ou 4 groupes méthyle ;

n est 2 ou 3 ;

$R_1$ est H et $R_2$ et $R_3$ forment ensemble un groupe $(CH_2)_p$ dans lequel p est 3 ou 4 ;

le cycle butyrolactone étant accroché au cycle pyridyle en position 2', 3' ou 4'

et les sels, isomères et mélanges d'isomères desdits produits, caractérisé en ce que l'on utilise comme produit de départ le pyridylacétonitrile anion que l'on fait réagir dans un solvant inerte et à basse température sur un époxyde de formule

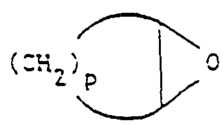

puis on réalise l'hydrolyse acide du produit hydroxylé obtenu et, enfin, on fait réagir le produit obtenu, en solution et en présence d'un agent de sodation, avec un réactif de formule

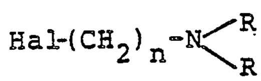 ,

dans laquelle Hal est un halogène ; le produit de formule (I) ainsi obtenu est éventuellement transformé en l'un de ses sels.

9. Procédé selon la revendication 8, caractérisé en ce que l'hydrure de sodium est employé comme agent de sodation.

10. Utilisation des dérivés de la γ-butyrolactone de formule (I) selon l'une des revendications 1 ou 8, pour la préparation de médicaments, présentant notamment un effet protecteur du myocarde.

11. Utilisation selon la revendication 10 pour la préparation de médicaments contenant de 5 à 800 mg desdits dérivés.

**Claims** (for the Contracting States : BE, CH, DE, FR, GB, IT, LI, LU, NL, SE)

1. Derivatives of γ-butyrolactone of formula :

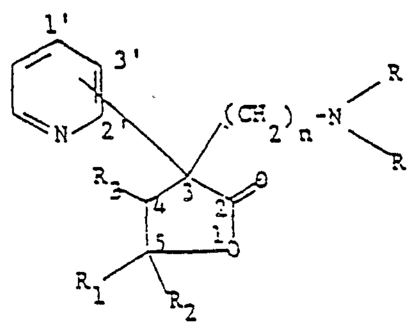

(I)

in which :

R is a straight or branched alkyl group having from 2 to 5 atoms of carbon, or the group

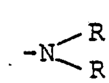

represents a morpholino or piperidino group possibly substituted by 1 to 4 methyl groups ;

n = 2 or 3 ;

$R_1$ and $R_2$ considered independently each represent hydrogen or an alkyl group having from 1 to 4 carbon atoms ; or $R_1$ and $R_2$ taken together represent a $(CH_2)_m$ group where m = 4 or 5, and $R_3$ is H or $R_1$ is H and $R_2$ and $R_3$ taken together represent $(CH_2)_p$ where p is 3 or 4,

the butyrolactone cycle being attached to the pyridyl cycle in 2', 3' or 4' position,

and the salts, isomers and mixtures of isomers of said products.

2. Derivatives according to claim 1, characterized in that $R_1$ and $R_2$ each represent the methyl group, $R_3$ is hydrogen, n = 2 and

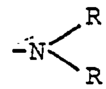

is the cis

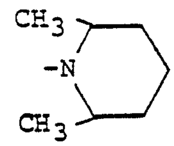

group, the butyrolactone group being attached to the pyridyl cycle in position 2′ or 3′.

3. Derivative according to claim 1, characterized in that $R_1$ and $R_2$ together represent the $-(CH_2)_5-$ group, $R_3$ is hydrogen, n = 2 and

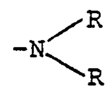

is the cis

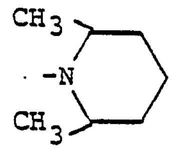

group, the butyrolactone group being attached to the pyridyl cycle in position 2′.

4. Derivative according to claim 1, characterized in that $R_1$ and $R_2$ each represent the ethyl group, $R_3$ is hydrogen, n = 2 and

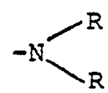

is the cis

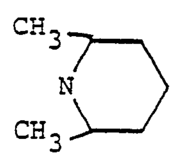

group, the butyrolactone being attached to the pyridyl cycle in position 2′.

5. A process for preparing the products according to any one of claims 1 to 4, of formula (I), in which $R_3$ is H, characterized in that it comprises the steps of using as starting product pyridylacetonitrile which is reacted on the compound of formula

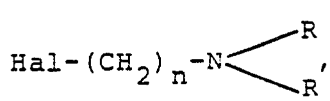 ,

in which Hal is a halogen, n and R having the meanings given in claim 1, the reaction being effected in the presence of an organic base, reacting the product obtained on a compound selected from the products of formula Hal—$CH_2$—$CH_2OA$, in which Hal is a halogen and A is a group protecting the hydroxyl function or of formula

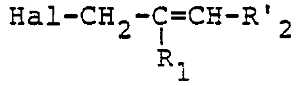 ,

in which $R_1$ is H or an alkyl group in $C_1$-$C_4$, or $R'_2$ is H or an alkyl group in $C_1$-$C_3$ or $R_1$ and $R'_2$ form together a group $-(CH_2)_{m'}$ in which m′ = 3 or 4, Hal is a halogen ; then effecting cyclization of the product obtained by a concentrated inorganic acid, and optionally, the compound of formula (I) thus obtained is converted into one of its salts.

6. The process according to claim 5, in which the reagent of the second step is Hal—$CH_2$—$CH_2$—OA, characterized in that this second step is effected in solution in an inert solvent, in the presence of sodium hydride, at a temperature of 80 to 120 °C.

7. The process according to claim 5, in which the reagent of the second step is

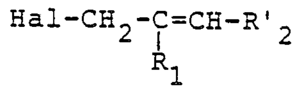

characterized in that the reaction is carried out in solution in a solvent in the presence of sodium amide.

8. Process according to claim 7, characterized in that the reaction is carried out in solution in dimethylformamide.

9. A process for preparing the products according to claim 1, of formula I, in which $R_1$ is H and $R_2$ and $R_3$ together represent the radical $(CH_2)_p$ in which p is 3 or 4, said process being characterized in that it comprises the steps of using as starting product the pyridylacetonitrile anion which is reacted in an inert solvent and at low temperature on an epoxide of formula

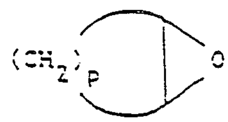

then in effecting acid hydrolysis of the hydroxylated product obtained, and finally reacting the product obtained in solution and in the presence of a sodation agent, with a reagent of formula

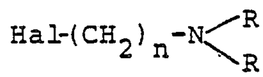 ,

in which Hal is a halogen ; the product of formula (I) thus obtained is then optionally converted into one of its salts.

10. Process according to claim 9, characterized in that sodium hydride is used as sodation agent.

11. Drugs having in particular a myocardium-protecting effect, characterized in that they contain at least one product according to any one of claims 1 to 4.

12. The drugs according to claim 11, characterized in taht they contain from 5 to 800 mg of said product according to any one of claims 1 to 4.

**Claims** (for the Contracting State AT)

1. Process for preparing derivatives of γ-butyrolactone of formula :

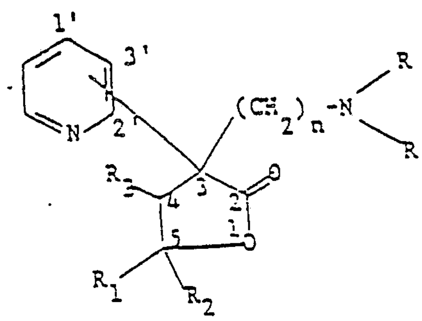 (I)

in which

R is a straight or branched alkyl group having from 2 to 5 atoms of carbon, or the group

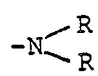

represents a morpholino or piperidino group possibly substituted by 1 to 4 methyl groups ;

n = 2 or 3 ;

$R_1$ and $R_2$ considered independently each represent hydrogen or an alkyl group having from 1 to 4 carbon atoms ; or $R_1$ and $R_2$ taken together represent a $(CH_2)_m$ group where m = 4 or 5, and $R_3$ is H ; the butyrolactone cycle being attached to the pyridyl cycle in 2', 3' or 4' position ;

and the salts, isomers and mixtures of isomers of said products, characterized in that it comprises the steps of using as starting product pyridylacetonitrile which is reacted on the compound of formula

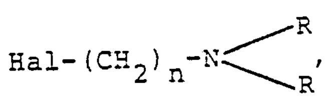 ,

in which Hal is a halogen, n and R having the above mentioned meanings, the reaction being effected in the presence of an organic base, reacting the product obtained on a compound selected from the products of formula Hal—$CH_2$—$CH_2OA$, in which Hal is a halogen and A is a group protecting the hydroxyl function, or of formula

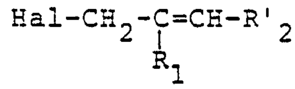

in which $R_1$ is H or an alkyl group in $C_1$-$C_4$, $R'_2$ is H or an alkyl group in $C_1$-$C_3$ or $R_1$ and $R'_2$ form a group —$(CH_2)_{m'}$ in which m' = 3 or 4, Hal is a halogen ; then effecting cyclization of the product obtained by a concentrated inorganic acid, and in that, optionally, the compound thus obtained of formula (I) is converted into one of its salts.

2. The process according to claim 1 in which the reagent of the second step is Hal—$CH_2$—$CH_2$—OA, characterized in that this second step is effected in solution in an inert solvent, in the presence of sodium hydride, at a temperature of 80 to 120 °C.

3. The process according to claim 1, in which the reagent of the second step is

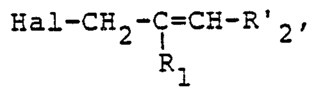

characterized in that the reaction is carried out in solution in a solvent, in the presence of sodium amide.

4. Process according to claim 3, characterized in that the reaction is carried out in dimethylformamide.

5. Process according to any one of claims 1 or 4, characterized in that it comprises the steps of reacting the 2-pyridyl or 3-pyridyl acetonitrile with a compound of formula

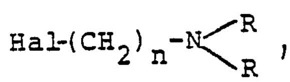

in which

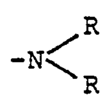

is the cis

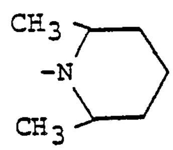

group, and n = 2 and in that the compound thus obtained is reacted with a compound of formula

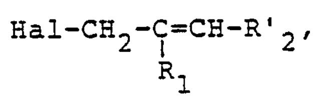

in which $R_1$ is $CH_3$ and $R'_2$ is hydrogen.

6. Process according to any one of claims 1 to 4, characterized in that the 2-pyridyl acetonitrile is reacted with a compound of formula

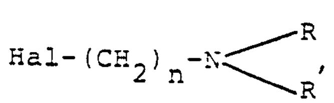

in which

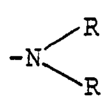

is the cis

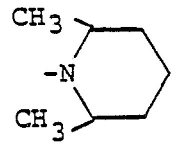

group and n = 2 and in that the compound thus obtained is reacted with a compound of formula

$$\mathrm{Hal{-}\underset{R_1}{C}{=}CH{-}R'_2},$$

in which $R_1$ and $R'_2$ form together the $-(CH_2)_4-$ group.

7. Process according to any one of claims 1 or 4, characterized in that the 2-pyridyl acetonitrile is reacted with a compound of formula

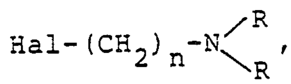

in which

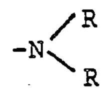

is the cis

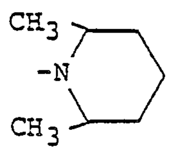

group, and n = 2 and in that the compound thus obtained is reacted with a compound of formula

$$\mathrm{Hal{-}CH_2{-}\underset{R_1}{C}{=}CH{-}R'_2},$$

in which $R_1$ is the ethyl group and $R'_2$ is the methyl group.

8. Process for preparing derivatives of γ-butyrolactone of formula (I) in which :

R is a straight or branched alkyl group having from 2 to 5 atoms of carbon, or the group

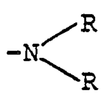

represents a morpholino or piperidino group possibly substituted by 1 or 4 methyl groups ;

n = 2 or 3 ;

$R_1$ is H and $R_2$ and $R_3$ taken together represent a $(CH_2)_p$ group in which p is 3 or 4 ;
the butyrolactone cycle being attached to the pyridyl cycle in 2', 3' or 4' position,
and the salts, isomers and mixtures of isomers of said products, characterized in that it comprises the steps of using as starting product pyridylacetonitrile anion which is reacted in an inert solvent and at low temperature on an epoxy of formula

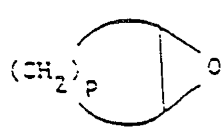

then in effecting acid hydrolysis of the resulting hydroxylated product, and finally reacting the product obtained in solution and in the presence of a sodation agent, with a reagent of formula

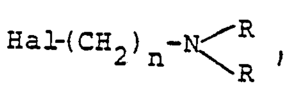

in which Hal is a halogen ; the product of formula (I) thus obtained is optionally converted into one of its salts.

9. Process according to claim 8, characterized in that sodium hydride is used as sodation agent.

10. Use of the derivatives of γ-butyrolactone of formula (I) according to any one of claims 1 or 8, for the preparation of drugs, having in particular a myocardium-protecting effect.

11. Use according to claim 10, for the preparation of drugs containing from 5 to 800 mg of said derivatives.

**Patentansprüche** (für die Vertragsstaaten : BE, CH, DE, FR, GB, IT, LI, LU, NL, SE)

1. γ-Butyrolacton-Derivate der Formel

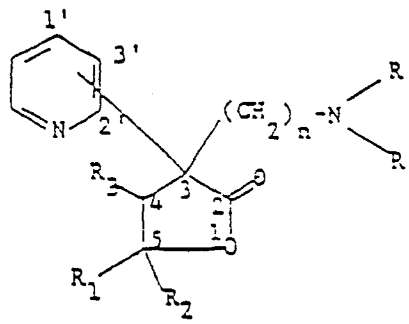

(I)

worin

R eine gerade oder verzweigte Alkylgruppe mit 2 bis 5 Kohlenstoffatomen ist oder die Gruppe

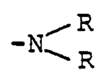

eine gegebenenfalls mit 1 bis 4 Methylgruppen substituierte Morpholino- oder Piperidinogruppe darstellt ;

n = 2 oder 3 ;

$R_1$ und $R_2$, einzeln betrachtet, jeweils Wasserstoff oder eine Alkylgruppe mit 1 bis 4 Kohlenstoffatomen darstellen ; oder $R_1$ und $R_2$, zusammen genommen, eine Gruppe $(CH_2)_m$ bedeuten, worin m = 4 oder 5, und $R_3$ für H steht, oder aber $R_1$ für H steht und $R_2$ und $R_3$, zusammen genommen, $(CH_2)_p$ darstellen, worin p 3 oder 4 ist ;

wobei der Butyrolactonring am Pyridylring in der Position 2', 3' oder 4' hängt,

und die Salzen, Isomeren und Isomerenmischungen dieser Produkte.

2. Derivat nach Anspruch 1, dadurch gekennzeichnet, daß $R_1$ und $R_2$ jeweils eine Methylgruppe darstellen, $R_3$ Wasserstoff ist, n = 2 und

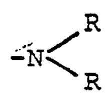

für die Gruppe

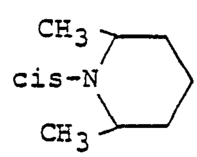

steht, wobei der Butyrolactonring am Pyridylring in der Position 2' oder 3' hängt.

3. Derivat nach Anspruch 1, dadurch gekennzeichnet, daß $R_1$ und $R_2$ zusammen die Gruppe $-(CH_2)_5-$ darstellen, $R_3$ Wasserstoff ist, n = 2 und

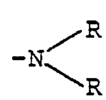

für die Gruppe

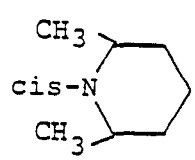

steht, wobei der Butyrolactonring am Pyridylring in der Position 2' hängt.

4. Derivat nach Anspruch 1, dadurch gekennzeichnet, daß $R_1$ und $R_2$ jeweils eine Äthylgruppe darstellen, $R_3$ Wasserstoff ist, n = 2 und

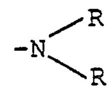

für die Gruppe

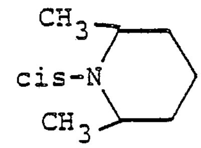

steht, wobei der Butyrolactonring am Pyridylring in der Position 2' hängt.

5. Verfahren zur Herstellung der Produkte nach einem der Ansprüche 1 bis 4 der Formel (I), worin $R_3$ für H steht, dadurch gekennzeichnet, daß man als Ausgangsprodukt Pyridylacetonitril verwendet, das man mit der Verbindung der Formel

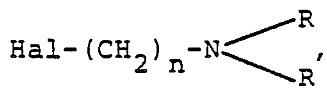

worin Hal ein Halogen ist, n und R die in Anspruch 1 angegebenen Bedeutungen haben, reagieren läßt, wobei die Reaktion in Gegenwart einer organischen Base durchgeführt wird ; daß man das erhaltene Produkt mit einer Verbindung, ausgewählt aus den Produkten der Formel Hal—$CH_2$—$CH_2$—OA, worin Hal ein Halogen ist und A eine Schutzgruppe der Hydroxylfunktion darstellt, oder der Formel

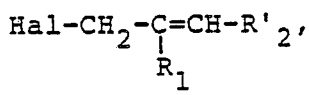

worin $R_1$ für H oder eine $C_1$-$C_4$-Alkylgruppe steht, $R'_2$ für H oder eine $C_1$-$C_3$-Alkylgruppe steht, oder $R_1$ und $R'_2$ zusammen eine Gruppe —$(CH_2)_{m'}$ bilden, worin m' = 3 oder 4, Hal ein Halogen ist, umsetzt ; man dann die Zyklisierung des erhaltenen Produkts mit einer konzentrierten Mineralsäure durchführt, und daß gegebenenfalls die so erhaltene Verbindung der Formel (I) in eines ihrer Salze übergeführt wird.

6. Verfahren nach Anspruch 5, bei welchem das Reagens der zweiten Stufe Hal—$CH_2$—$CH_2$—OA ist, dadurch gekennzeichnet, daß diese zweite Stufe in Lösung in einem inerten Lösungsmittel in Gegenwart von Natriumhydrid bei einer Temperatur von 80 bis 120 °C durchgeführt wird.

7. Verfahren nach Anspruch 5, bei welchem das Reagens der zweiten Stufe

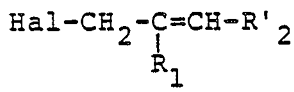

ist, dadurch gekennzeichnet, daß die Reaktion in Lösung in einem Lösungsmittel in Gegenwart von Natriumamid durchgeführt wird.

8. Verfahren nach Anspruch 7, dadurch gekennzeichnet, daß die Reaktion in Lösung in Dimethylformamid durchgeführt wird.

9. Verfahren zur Herstellung der Produkte nach Anspruch 1 der Formel I, worin $R_1$ für H steht und $R_2$ und $R_3$ zusammen die Gruppe $(CH_2)_p$ darstellen, worin p 3 oder 4 ist, dadurch gekennzeichnet, daß man als Ausgangsprodukt Pyridylacetonitril-Anion verwendet, das man in einem inerten Lösungsmittel und bei niedriger Temperatur mit einem Epoxid der Formel :

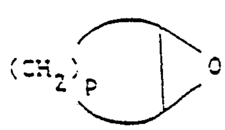

reagieren läßt, man dann die saure Hydrolyse des erhaltenen hydroxylierten Produkts durchführt, und man schließlich das erhaltene Produkt in Lösung und in Gegenwart eines Natrisierungsmittels mit einem Reagens der Formel

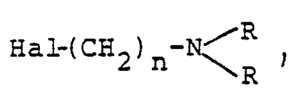

worin Hal ein Halogen ist, zur Umsetzung bringt, wobei das so erhaltene Produkt der Formel (I) gegebenenfalls in eines seiner Salze übergeführt wird.

10. Verfahren nach Anspruch 9, dadurch gekennzeichnet, daß Natriumhydrid als Natrisierungsmittel eingesetzt wird.

11. Medikamente, die insbesondere Myokardschutzwirkung haben, dadurch gekennzeichnet, daß sie mindestens ein Produkt nach einem der Ansprüche 1 bis 4 enthalten.

12. Medikamente nach Anspruch 11, dadurch gekennzeichnet, daß sie 5 bis 800 mg des Produkts nach einem der Ansprüche 1 bis 4 enthalten.

**Patentansprüche** (für den Vertragsstaat AT)

1. Verfahren zur Herstellung von γ-Butyrolacton-Derivaten der Formel

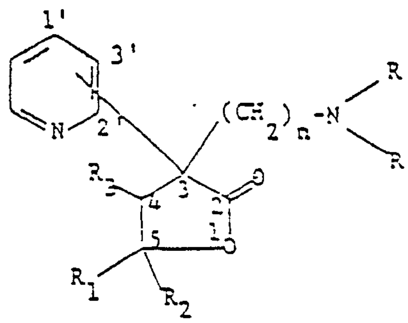

worin

R eine gerade oder verzweigte Alkylgruppe mit 2 bis 5 Kohlenstoffatomen ist, oder die Gruppe

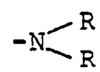

eine gegebenenfalls mit 1 bis 4 Methylgruppen substituierte Morpholino- oder Piperidinogruppe darstellt ;

n = 2 oder 3 ;

$R_1$ und $R_2$, einzeln betrachtet, jeweils Wasserstoff oder eine Alkylgruppe mit 1 bis 4 Kohlenstoffatomen darstellen ; oder $R_1$ und $R_2$, zusammen genommen, eine Gruppe $(CH_2)_m$ bedeuten, worin m = 4 oder 5, und $R_3$ für H steht ;

wobei der Butyrolactonring am Pyridylring in der Position 2', 3' oder 4' hängt,

und der Salze, Isomeren und Isomerenmischungen dieser Produkte, dadurch gekennzeichnet, daß man als Ausgangsprodukt Pyridylacetonitril verwendet, das man mit der Verbindung der Formel

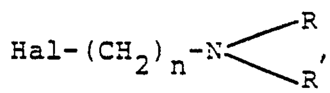

worin Hal ein Halogen ist, n und R die oben angegebenen Bedeutungen haben, reagieren läßt, wobei die Reaktion in Gegenwart einer organischen Base durchgeführt wird ; daß man das erhaltene Produkt mit einer Verbindung, ausgewählt aus den Produkten der Formel Hal—$CH_2$—$CH_2$—OA, worin Hal ein Halogen ist und A eine Schutzgruppe der Hydroxylfunktion darstellt, oder der Formel

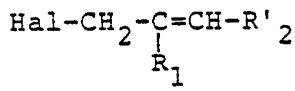

worin $R_1$ für H oder eine $C_1$-$C_4$-Alkylgruppe steht, $R'_2$ für H oder eine $C_1$-$C_3$-Alkylgruppe steht, oder $R_1$ und $R'_2$ zusammen eine Gruppe —$(CH_2)_{m'}$ bilden worin m' = 3 oder 4, Hal ein Halogen ist, umsetzt ; man dann die Zyklisierung des erhaltenen Produkts mit einer konzentrierten Mineralsäure durchführt, und daß gegebenenfalls die so erhaltene Verbindung der Formel (I) in eines ihrer Salze übergeführt wird.

2. Verfahren nach Anspruch 1, bei welchem das Reagens der zweiten Stufe Hal—$CH_2$—$CH_2$—OA ist, dadurch gekennzeichnet, daß diese zweite Stufe in Lösung in einem inerten Lösungsmittel in Gegenwart von Natriumhydrid bei einer Temperatur von 80 bis 120 °C durchgeführt wird.

3. Verfahren nach Anspruch 1, bei welchem das Reagens der zweiten Stufe

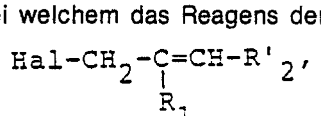

ist, dadurch gekennzeichnet, daß die Reaktion in Lösung in einem Lösungsmittel in Gegenwart von Natriumamid durchgeführt wird.

4. Verfahren nach Anspruch 3, dadurch gekennzeichnet, daß die Reaktion in Lösung in Dimethylformamid durchgeführt wird.

5. Verfahren nach einem der Ansprüche 1 oder 4, dadurch gekennzeichnet, daß man 2-Pyridyl- oder 3-Pyridyl-acetonitril mit einer Verbindung der Formel

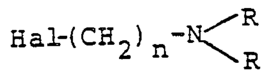,

worin

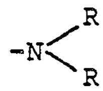

für die Gruppe

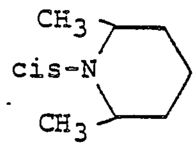

steht und n = 2, reagieren läßt, und daß man die erhaltene Verbindung mit einer Verbindung der Formel

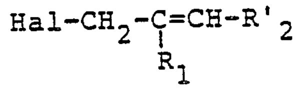,

worin $R_1$ $CH_3$ ist und $R'_2$ Wasserstoff bedeutet, reagieren läßt.

6. Verfahren nach einem der Ansprüche 1 oder 4, dadurch gekennzeichnet, daß man 2-Pyridyl-acetonitril mit einer Verbindung der Formel

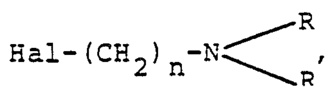,

worin

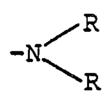

für die Gruppe

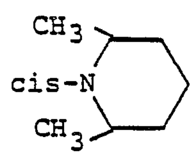

steht und n = 2, reagieren läßt, und daß man die erhaltene Verbindung mit einer Verbindung der Formel

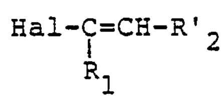,

worin $R_1$ und $R'_2$ zusammen die Gruppe $-(CH_2)_4-$ bilden, reagieren läßt.

7. Verfahren nach einem der Ansprüche 1 oder 4, dadurch gekennzeichnet, daß man 2-Pyridyl-acetonitril mit einer Verbindung der Formel

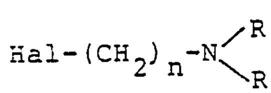,

worin

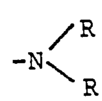

für die Gruppe

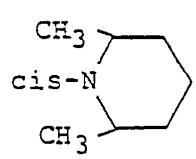

steht und n = 2, reagieren läßt, und daß man die erhaltene Verbindung mit einer Verbindung der Formel

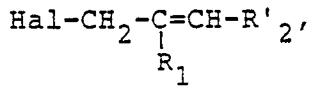

worin $R_1$ die Gruppe Äthyl und $R_2$ die Gruppe Methyl ist, reagieren läßt.

8. Verfahren zur Herstellung von γ-Butyrolacton-Derivaten der Formel (I), worin :

R eine gerade oder verzweigte Alkylgruppe mit 2 bis 5 Kohlenstoffatomen ist, oder die Gruppe

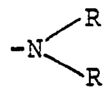

eine gegebenenfalls mit 1 oder Methylgruppen substituierte Morpholino- oder Piperidinogruppe darstellt ;

n = 2 oder 3 ;

$R_1$ für H steht und $R_2$ und $R_3$ zusammen eine Gruppe $(CH_2)_p$ bilden, worin p 3 oder 4 ist ;

wobei der Butyrolactonring am Pyridylring in der Position 2', 3' oder 4' hängt,

und der Salze, Isomeren und Isomerenmischungen dieser Produkte dadurch gekennzeichnet, daß man als Ausgangsprodukt Pyridylacetonitril-Anion verwendet, das man in einem inerten Lösungsmittel und bei niedriger Temperatur mit einem Epoxid der Formel

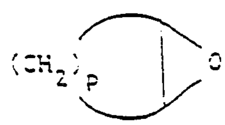

reagieren läßt, man dann die saure Hydrolyse des erhaltenen hydroxylierten Produkts durchführt, und man schließlich das erhaltene Produkt in Lösung und in Gegenwart eines Natrisierungsmittels mit einem Reagens der Formel

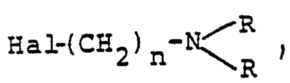

worin Hal ein Halogen ist, zur Umsetzung bringt ; wobei das so erhaltene Produkt der Formel (I) gegebenenfalls in eines seiner Salze übergeführt wird.

9. Verfahren nach Anspruch 8, dadurch gekennzeichnet, daß Natriumhydrid als Natrisierungsmittel eingesetzt wird.

10. Verwendung der γ-Butyrolacton-Derivate der Formel (I) nach einem der Ansprüche 1 oder 8 zur Herstellung von Medikamenten, die insbesondere Myokardschutzwirkung haben.

11. Verwendung nach Anspruch 10 zur Herstellung von Medikamenten, die 5 bis 800 mg der genannten Derivate enthalten.